(19) Europäisches Patentamt
European Patent Office
Office européen des brevets

(11) **EP 3 777 667 A1**

(12) **EUROPEAN PATENT APPLICATION**

(43) Date of publication:
**17.02.2021 Bulletin 2021/07**

(51) Int Cl.:
***A61B 5/026*** (2006.01)

(21) Application number: **19461568.8**

(22) Date of filing: **16.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Angionica Sp. z o.o.**
**90-924 Lódz (PL)**

(72) Inventors:
• **GEBICKI, Jerzy**
  **90-010 Lodz (PL)**
• **MARCINEK, Andrzej**
  **91-496 Lodz (PL)**

(74) Representative: **Patpol Kancelaria Patentowa Sp. z o.o.**
**Nowoursynowska 162J**
**02-776 Warszawa (PL)**

(54) **A METHOD OF DETECTION OF THE PRESENCE OF A PATHOLOGY THAT IMPAIRS BLOOD MICROCIRCULATION OSCILLATIONS IN A WOMAN AND DEVICE THEREFOR**

(57) A method of detection of the presence of a pathology that impairs blood microcirculation or a risk of said pathology, in a female human subject of the age below 60 years, by determination of a parameter of oscillatory function of the skin microcirculation in said female subject, said method comprising the following steps: - illumination of a selected location on non-hairy skin of said female human subject in a resting steady condition without any blockage or stimulation of the blood flow with exciting light capable of inducing skin NADH fluorescence signal for a period of time; - simultaneously with said illumination detecting, measuring and recording as a function of time an induced NADH fluorescence signal emitted from the said selected location to obtain the time course of the NADH fluorescence signal intensity in the said period of time; and - computer implemented steps of: - fitting of a baseline about which the NADH fluorescence signal oscillates to the said time course of the NADH fluorescence signal intensity; and - determination of a mean squared error of the deviation of the NADH fluorescence signal from the fitted baseline as the parameter of oscillatory function of skin microcirculation; a method of determination of said parameter of oscillatory function of the skin microcirculation as well as device or system therefor.

Fig. 1

## Description

Field of the invention

The invention relates to a method of detection of the presence of a pathology that impairs blood microcirculation oscillations (flowmotion) in a female human subject of the age below 60 years, a method of determination of a parameter for said detection, as well as a system or device for performing said detection or determination.

Prior art

Natural and spontaneous phenomenon observed in the vascular networks both in vitro and in vivo is so called vasomotion, i.e. rhythmic oscillations of vascular tone that is generated within a vascular wall. Vasomotion is caused by local changes in smooth muscle constrictions and dilations and is not a consequence of heartbeat, respiration or neuronal input (H. Nillson, C. Aalkjær., Vasomotion: Mechanisms and Physiological Importance, Molecular Interventions 3(2), 79-89 (2003)). Vasomotion is responsible for rhythmic variations (oscillations) of blood flow pressure and flow, also called flowmotion.

Microcirculation is the part of the circulation system having very important physiological function. Microcirculation vessels ensure efficient diffusion-limited exchange of gases, metabolites and elements of the immune system between blood and extravascular aqueous space as well as efficient humoral and thermal regulation.

Dysfunction and impairment of microcirculation play a key role in the pathophysiology of many diseases. Microvascular changes were also observed in hypertension. See H.Nillson, C. Aalkjær, Vasomotion: Mechanisms and Physiological Importance, Molecular Interventions, 3(2), 79-89 (2003); B. Gryglewska, Znaczenie mikrokrążenia w nadcisnieniu tętniczym i mozliwosci jego oceny na podstawie badania mikrounaczynienia skórnego, Nadcisnienie Tętnicze, 14(5), 395-410 (2010). Microcirculation is impaired in such pathologies as cardiovascular diseases, including coronary artery disease and stroke, diabetes, including type 2 diabetes, metabolic syndrome, cancer, and autoimmunological diseases. Both males and post-menopausal females are at similar enhanced and great risk of such pathologies. See V. L. Clifton et al., Microvascular effects of corticotropin-releasing hormone in human skin vary in relation to estrogen concentration during the menstrual cycle, Journal of Endocrinology 186, 69-76 (2005). Microvascular dysfunction and impairment may play a central role by increasing not only peripheral vascular resistance and blood pressure (BP), but also by decreasing insulin-stimulated glucose uptake in target cells. This metabolic insulin resistance is functionally coupled with insulin vascular resistance via shared insulin signaling pathways in the endothelium and skeletal muscles. See A. Vinet et al., Impact of a Lifestyle Program on Vascular Insulin Resistance in Metabolic Syndrome Subjects: The RESOLVE Study, The Journal of Clinical Endocrinology and Metabolism, 100(2):442-450 (2015).

As skin microcirculatory investigation can mirror the state of microcirculation in other vascular beds, the human skin circulation has been used as a non-invasive model for the examination of peripheral vascular function. Therefore, microcirculation is mostly studied in humans at the level of skin, i.e. as a skin microcirculation. Although rhythmic motions of vessel wall and triggered by them changes of blood flow are seen in the whole vascular bed, their visualization and recording are especially easy in the vasculature of the skin. Skin microcirculation vasomotion is the rhythmic variation of the skin microvessel diameter and is responsible for the skin microcirculatory blood flow oscillations, i.e. the skin blood flowmotion. Functional impairment (dysfunction) of the microcirculatory function of many regions, tissues and organs, including impairment of natural and spontaneous blood flow oscillations plays very important role in pathophysiology of many diseases, especially such as chronic renal failure, vascular diseases and hypertension. As skin microcirculation impairment mirrors impairments in other regions, the analysis of microcirculatory blood flow oscillations or the skin blood flowmotion has a diagnostic value (Stewart, J., et al., Noninvasive interrogation of microvasculature for signs of endothelial dysfunction in patients with chronic renal failure, American Journal of Physiology - Heart and Circulatory Physiology 287, H2687-H2696 (2004).

Microcirculatory blood flow has been up to now investigated indirectly in humans at the level of skin by means of laser Doppler flowmetry/fluximetry (LDF) and spectral Fourier analysis (transformation) of skin LDF signal from flow/flux variations in the time domain into frequency spectrum (M. Rossi, Diagnostic values of skin vasomotion investigation in vascular diseases, Advances in Biomedical Research, 374-380 (2010). Assessment of microcirculatory function by LDF is performed both under resting conditions and during flow changes induced by ischemia, heating or administration of vasoactive substances.

Other optical methods like photoplethysmography (PPG), pulse oximetry, and diffuse reflection spectroscopy rely on the blood absorption and reflection of light by blood to estimate the blood volume and oxygen saturation. They give no information about the blood flow.

LDF method is virtually the only existing method of non-invasive investigation of microcirculation and is based on the utilization of the Doppler shift arising when the laser light is scattered by a red cell moving in the microvessels. However, it has certain disadvantages, i.e. high cost, uncertainty and variability of obtained measurements, troublesome intra- and interindividual comparison.

Often, dysfunction and impairment of blood microcirculation occurs very early, before any physical or macroscopic signs of a pathology that impairs microcirculation are present and can be observed and detected. Impairment of microcirculation can be used for early detection of the presence of such a pathology or risk thereof before any physical or macroscopic signs of the pathology are present.

A testing method is needed that would allow to investigate and assess microcirculation flowmotion (oscillations) function and detect any dysfunctions of microcirculation flowmotion at an early stage of impairment in order to identify patients for further more detailed and complicated diagnostic tests towards the identification of the type of pathology and/or for prophylactic or therapeutic intervention.

The need exists to provide a non-invasive test for investigating and assessing microcirculation flowmotion (oscillations) function which would be reliable, easy to carry out and inexpensive, and capable of objective standardization and thus applicable for tests in large patient populations, for example for screening purposes.

There is also a need for a simple, quick and non-expensive test that would allow to monitor and control the response of a patient to a medical treatment of a pathology.

It has now been found that measurement of skin NADH fluorescence in resting conditions, that is without any mechanical, physical or pharmacological stimulation or blockage and release of blood flow can be successfully used for the assessment of spontaneous skin microcirculation oscillations (flowmotion) and in consequence detection of the presence of pathology that impairs blood microcirculation or risk thereof.

Summary of the invention.

There is provided a method of detection of the presence of a pathology that impairs blood microcirculation or risk of said pathology, in a female human subject of the age below 60 years, by determination of a parameter of oscillatory function of skin microcirculation in said female human subject, said method comprising the following steps:

- illumination of a selected location on non-hairy skin of said female human subject in a resting steady condition without any blockage or stimulation of the blood flow with exciting light capable of inducing skin NADH fluorescence signal for a period of time;

- simultaneously with said illumination detecting, measuring and recording as a function of time an induced NADH fluorescence signal emitted from the said selected location to obtain the time course of the NADH fluorescence signal intensity in the said period of time; and

- computer implemented steps of:

  - fitting of a baseline about which the NADH fluorescence signal oscillates to the said time course of the NADH fluorescence signal intensity; and
  - determination of a mean squared error of the deviation of the NADH fluorescence signal from the fitted baseline as the parameter of oscillatory function of skin microcirculation.

There is also provided a method of determination of a parameter for detection of the presence of a pathology that impairs blood microcirculation or risk of said pathology, in a female human subject of the age below 60 years, and a device or system therefor.

Definition of the parameter of oscillatory function of skin microcirculation in the method of the invention as the mean squared error of the deviation of the NADH fluorescence signal from the fitted baseline makes this parameter an objective, operator-independent, and patient-specific result of the test. Such a characteristic of the parameter allows its easy standardization.

Time-course of NADH fluorescence intensity signal allows to obtain important data for analysis of microcirculation dysfunctions.

Brief description of the Figures of Drawing

Figs. 1 to 4 show recorded time courses of flow-mediated skin NADH fluorescence and fitted baselines in apparently healthy human subject - volunteers.

Figs. 5 to 10 show recorded time courses of flow-mediated skin NADH fluorescence and fitted baselines in patients.

Fig. 11 shows correlation of $FM_{index}$ with age for control group.

Fig. 12 shows comparison of $FM_{index}$ for control group with respect to sex and age.

Fig. 13 shows comparison of $FM_{index}$ for group of diabetic patients DM1 with respect to sex and age.

Fig. 14 shows comparison of $FM_{index}$ for group of diabetic patients DM2 with respect to sex and age.

Fig. 15 shows ROC (AUC) curves for female diabetic patients in groups DM1 and DM2.

Fig. 16 shows ROC (AUC) curves for male diabetic patients in groups DM1 and DM2.

Detailed description of the invention.

The present invention provides a method of detection of the presence of a pathology that impairs blood microcirculation or a risk of said pathology, in a female human subject of the age below 60 years, by determination of a parameter of oscillatory function of skin microcirculation in said female human subject, said method comprising the following steps:

- illumination of a selected location on non-hairy skin of said female human subject in a resting steady condition without any blockage or stimulation of the blood flow with exciting light capable of inducing skin NADH fluorescence signal for a period of time;

- simultaneously with said illumination detecting, measuring and recording as a function of time an induced NADH fluorescence signal emitted from the said selected location to obtain the time course of the fluorescence signal intensity in the said period of time; and

- computer implemented steps of:

   - fitting of a baseline about which the NADH fluorescence signal oscillates to the said time course of the fluorescence signal intensity; and

   - determination of a mean squared error of the deviation of the NADH fluorescence signal from the fitted baseline as said parameter of oscillatory function of the skin microcirculation.

The present invention provides also a method of determination of a parameter for detection of the presence of a pathology that impairs blood microcirculation, or a risk of said pathology, in a female human subject of the age below 60 years, wherein said parameter is determined as a parameter of oscillatory function of skin microcirculation in the following steps:

- illumination of a selected location on non-hairy skin of said female human subject in a resting steady condition without any blockage or stimulation of the blood flow with exciting light capable of inducing skin NADH fluorescence signal for a period of time;

- simultaneously with said illumination detecting, measuring and recording as a function of time an induced NADH fluorescence signal emitted from the said selected location to obtain the time course of the fluorescence signal intensity in the said period of time; and

- computer implemented steps of:

   - fitting of a baseline about which the NADH fluorescence signal oscillates to the said time course of the fluorescence signal intensity; and

   - determination of a mean squared error of the deviation of the NADH fluorescence signal from the fitted baseline as the parameter of oscillatory function of skin microcirculation.

In another aspect the present invention provides also a device or system for determination of a parameter for detection of the presence of a pathology that impairs blood microcirculation, or a risk of said pathology, in a female human subject of the age below 60 years, said parameter being determined as a parameter of oscillatory function of skin microcirculation, the device or system consisting of:

- a means for illumination of a skin of said female human subject with exciting light capable to induce NADH fluorescence;

- a means for detecting and measuring NADH fluorescence signal emitted from the skin;

- a processing unit which is configured to perform the following:

  - receiving and recording time course of said intensity of the NADH fluorescence signal,

  - fitting of a baseline about which the NADH fluorescence signal oscillates to the said time course of the fluorescence signal, and

  - determination a mean squared error of the deviation of the NADH fluorescence signal from the fitted baseline as the parameter of oscillatory function of skin microcirculation,

  and

- a means for displaying a result of determination of said parameter of oscillatory function of skin microcirculation.

The device or system according to the present invention may further comprise a means for displaying the fluorescence signal oscillations against time and its pattern analysis or for retrieving and saving the said course of the fluorescence signal against time for it to be printed and its pattern analyzed later.

There are many pathologies that impair blood microcirculation. In particular, pathology that impairs blood microcirculation can be selected from the group consisting of diabetes and its complications, including diabetes mellitus type 1 and diabetes mellitus type 2, metabolic syndrome, cardiovascular disease, including coronary arterial disease, idiopathic hypertension, myocardial infarct and stroke, cancer, neurodegenerative diseases, autoimmunological diseases, depression, chronic renal failure and bad abidance of menopause.

Said pathologies highly influence and impair blood microcirculation at the very early stage of disease, often earlier than any other signs, such as macroscopic signs, can be observed. For example, EGFR marker (Epidermal Growth Factor Receptor) that highly influences flowmotion, is raised in breast cancer 18 months before macroscopic change emerges. Therefore, analysis of the flowmotion parameter can turn out to be a better marker of the breast cancer than mammography.

In one embodiment, said detection is therefore an early detection.

Neurodegenerative disease in particular is amyotrophic lateral sclerosis, Parkinon's disease, Alzheimer's disease and Huntington disease.

Autoimmunological disease in particular is systemic lupus erythematosus, rheumatoid arthritis, multiple sclerosis, psoriasis and inflammatory bowel disease.

In a preferred embodiment, said pathology that impairs blood microcirculation is selected from the group consisting of diabetes mellitus, in particular diabetes mellitus 1 and diabetes mellitus 2, diabetic complications selected from the group consisting of retinopathy, neuropathy, neuropathy and diabetic foot, metabolic syndrome and cancer.

In another preferred embodiment said pathology that impairs blood microcirculation is selected from the group consisting of diabetes mellitus, in particular diabetes mellitus 1 and diabetes mellitus 2, and diabetic complications selected from the group consisting of retinopathy, neuropathy, neuropathy and diabetic foot.

In another preferred embodiment said pathology that impairs blood microcirculation is cancer, in particular breast cancer.

In another preferred embodiment said pathology that impairs blood microcirculation is neurodegenerative disease, in particular amyotrophic lateral sclerosis, Parkinson's disease, Alzheimer's disease and Huntington disease.

In another preferred embodiment said pathology that impairs blood microcirculation is autoimmunological disease, in particular systemic lupus erythematosus, rheumatoid arthritis, multiple sclerosis, psoriasis and inflammatory bowel disease.

In another preferred embodiment said pathology that impairs blood microcirculation is cardiovascular disease, in particular idiopathic hypertension, coronary arterial disease and stroke.

In another preferred embodiment said pathology that impairs blood microcirculation is chronic renal failure.

The present invention is based on unexpected finding that oscillatory function of blood microcirculation and the parameter thereof determined as skin microcirculation parameter in accordance with the method od the invention as defined above highly depends on the age in the group of females while depends very little, if at all, on the age in the group of males. At the age above 60 years essentially there is no difference between healthy males and females. However, it has been shown that in the group of females at the age 30-50 years observed values of the said parameter are ca. 2-times higher than in the group of males. This difference results from the powerful positive role of estrogen on microcirculation in females during pre-menopausal period. The role of testosterone in males is much weaker and age-dependence virtually does not exist.

It has been also found that type 1 diabetes removes any differences between female and male subjects with respect to the said parameter. This means that in type 1 diabetes the amount of microvascular complications is much greater in the females group than in males group.

The terms "human female subject", "woman", "female" and "female subject" can be used interchangeably herein.

The term "early detection" as used herein refers to detection of a pathology or risk thereof before any physical or macroscopic signs of the pathology are present and can be physically observed.

The term "oscillatory function of skin microcirculation" (that can be also called flowmotion) is to be understood in accordance with its accepted meaning in the art as the "natural" (spontaneous, at rest) oscillatory function, without any prior stimulation or blockage of the blood flow, either by physical or pharmacological means.

The method of the invention utilizes so-called flow-mediated skin fluorescence. The method is known, for example from M. Hellman et al., Microvascular Research, Reproducibility of flow mediated skin fluorescence to assess microvascular function. 113, 60-84 (2017); L. Piotrowski et al., Flow mediated skin fluorescence - A novel technique for evaluation of cutaneous microcirculation,Review of Scientific Instruments, 87(3): 036111 (2016); M. Tarnawska et el., A pilot study with flow mediated skin fluorescence: A novel device to assess microvascular endothelial function in coronary artery disease, Cardiology Journal, 25(2), 120-127 (2018); and WO2012/164494 in the application for determination of certain quantitative parameters of changes of skin NADH fluorescence signal mediated by blocking (ischemia) and then releasing (hyperemia) the blood flow in the artery. Ischemic (low flow) and hyperemic (high flow) responses give the information about the condition of vascular endothelium. Different ischemic and hyperemic responses were obtained between healthy subjects and patients with cardiovascular disease (CAD), a population with well characterized microvascular dysfunction. Oscillations of the time-course of skin NADH fluorescence as such were neither considered nor determined in the above method.

Oscillations of the time-course of skin NADH fluorescence in the method of the invention mirror the skin microcirculatory blood flow oscillations.

The data obtained in the method of the invention can be used in the early diagnostics of microcirculation dysfunction.

The method of the invention comprises illumination of a non-hairy skin surface of a female human subject in one selected location. Said non-hairy skin surface means inter alia the surface of the upper limb of the subject, such as forearm, hand or a finger, preferably the forearm. Such a non-hairy skin surface includes also the surface of the lower limb, such as thigh and feet. Such a non-hairy skin surface includes also for example the forehead area.

However, as the method requires measurement of skin fluorescence signal in a resting conditions and steady motionless state of the subject (patient), the most preferred location of illumination is the forearm, as it allows that patient be seated comfortably motionless during the test.

Illumination in one selected location should be understood as illumination wherein illumination means is directed onto one single position/location on the surface of the skin and is not intentionally moved with respect to this position/location.

It will be appreciated by a skilled person that flow-mediated skin fluorescence signal is emitted from the skin when the skin is illuminated with exciting light and during such illumination.

It should be understood that any mention herein of a fluorescence signal relates to the NADH fluorescence signal emitted by the skin of the subject.

It will be appreciated by a skilled person that exciting light capable of inducing NADH fluorescence will have the wavelength at the UV range absorbed by NADH. NADH absorbs light at almost the entire UVA spectrum in the range from 300 to 400 nm and in response to illumination emits fluorescence light at the range of 400 nm to 600 nm, with a maximum at about 460 nm.

Preferably, exciting light will have the wavelength at about 350 to 360 nm, especially 360 nm.

Preferably, emitted fluorescence light will be detected and measured at the wavelength in the range from 420 nm to 480 nm, especially about 460 nm.

The period of time during which the detection, measurement and recording of the fluorescence signal is performed is the period that allows to obtain sufficient evaluation of the fluorescence changes for computer implemented fitting of a baseline. Such sufficient period of time generally should be at least one minute, such as 1 to 5 minutes, such as 1, 2, 3, 4 or 5 minutes. Period of measurement can be longer than 5 minutes, such as up to 10 minutes.

Preferably, results of measurement are normalized with respect to the mean value of the fluorescence signal in the central period of measurement.

It should be understood that for performing computer implemented steps as defined above a computer program is used. It is advantageous to reject initial section of the measurement. For example, if the period of time of measurement is 5 minutes, then initial 1-minute section is rejected and period of measurement entailing second to fifth minutes, inclusive, is taken for fitting the baseline and determination of the mean squared error.

It should be understood that fitted baseline about which NADH fluorescence signal oscillates is an artificially generated/created line. Fitting of a baseline about which NADH fluorescence signal oscillates can be performed using polynomial regression method.

On the basis of the courses of flow-mediated skin fluorescence collected for the group of patients and a control group it has been found that a baseline about which the fluorescence signal oscillates is not horizontal and can have various patterns, i.e. can be an ascending, descending, ascending reaching plateau, descending reaching plateau, parabolic, etc., one.

Preferably, on the basis of such courses of a baseline second order polynomial regression method is used for fitting the baseline since it has the best fit to the changes in the course of fluorescence signal not related to the blood flow oscillations.

Polynomial, preferably second order polynomial is fitted to experimental points using linear regression method (least squares method). Such fitting is unique, as a result of the solution of the system of equations. For parabola it is a system of 3 equations that allow to find a unique parameters of the equation $y = intercept + B1*x + B2*x^2$.

In the least squares method the best fit is considered the one wherein sum of squares of deviations of the experimental point from polynomial function, i.e. $SSE = \sum(yi-Yi)^2$ (residual sum of squares) is minimal (yi - experimental points, Yi - corresponding points on the fitted curve). At the same time, SSE value is the measure of the fitting error (deviation of the points from polynomial relationship). In the case of analysis of oscillations, deviation of the fluorescence signal can be used as an assessment of oscillations measure; a parameter that is objective and patient specific result of the test.

In statistical analysis squared deviation (residual sum of squares) can be normalized with respect to the number of analyzed experimental points (the time of measurement of oscillations taken for analysis). That is, the mean squared error is calculated.

Mean squared error is defined as:

$$MSE = SSE/(n-m),$$

where

SSE is the residual sum of squares

n is the number of measurement points taken for analysis (it depends on the time interval and sampling frequency),

m is the number of regression parameters describing polynomial (3 in the case of second order polynomial).

MSE is unbiased estimator of deviation of the points/signals from the polynomial relationship. This allows comparison of the results obtained from observation of oscillations in different time intervals.

Defining oscillations parameter as MSE makes it objective, operator independent, characteristic for a patient and his testing with the method of flow-mediated skin fluorescence.

The present inventors have chosen to call the parameter thus defined in the tests described in the Examples that follow a Flowmotion index ($FM_{index}$). However, it is not limiting as it may be given any other name.

It will be appreciated by a skilled person that $FM_{index}$ as a parameter of oscillatory function has lower value when dysfunction or impairment of microcirculation is present in a given subject, compared with a subject with non-impaired microcirculation. The lower is the $FM_{index}$, the bigger is dysfunction or impairment of microcirculation. Measurements performed on bigger populations of subjects can be used to establish limiting values or respective standards.

It will be also appreciated that $FM_{index}$ as a parameter of oscillatory function does not allow as such to differentiate between specific types of pathologies underlying impairment of oscillatory function. However, it can be used as indicia that some type of such pathology or risk thereof can be present and serve for early detection or confirmation of the presence thereof.

The present invention provides also a device or system for performing the method of the invention as defined above.

Techniques of obtaining, detecting, measuring and recording NADH fluorescence signal from tissue cells (NADH fluorometry) as well as fluorometers are as such well known in the art and can be used to perform the method of the invention and in the device/system of the invention.

Said means for illuminating, detecting, measuring, recording and performing computer-implemented steps can be integrated in such a fluorometer.

Said illumination means will be a source of excitation light capable of emitting UV light in the range absorbed by NADH, i.e. at the wavelength range of 300 to 400 nm.

Said means for detecting will be a detector of fluorescence signal at least in the range emitted by NADH, i.e. at the wavelength range of 400 to 600 nm, preferably 420 nm to 480 nm, especially about 460 nm.

Said illumination and detecting means preferably are combined in a single measuring head to be placed at the selected location close to the skin of the subject. Such a measuring head will also have measurement window and can also comprise interference filters.

Generally, there is a certain flexibility in configuration of the device, provided that all essential elements listed above are included.

Conventional light sources of excitation light known in the art can be used, including filtered spectral lamp such as mercury or xenon lamp, light emitting diode LED, laser diode or pulsed laser. Advantageous light source is the light emitting diode LED.

Conventional detectors can be used, such as photodiode detector, fast photodiode detector, photon multiplier tube, etc.

The exciting light can be carried out to the skin through the window of the measuring head placed close to the skin, either in direct contact with the skin or in close vicinity to the skin. The emitted skin fluorescence can be collected through the same window of the measuring head.

The measuring head can be connected to the light source and detector through the light guide or it can comprise of both light source and detector in one arrangement.

Any conventional light guide can be used for carrying excitation light and for collecting fluorescence light, such as optical fibers, optionally in a flexible housing.

In one embodiment, the measurement will be performed on a forearm or the palmar side of a hand and the measuring head will be attached to the support for placing hand or to the band fixed on the hand.

In another embodiment, the measurement will be performed on the dorsal side of a hand placed on a support, such as tripod, and the measuring head will be fixed above the hand.

In another embodiment, the measurement will be performed on a finger, by means of a cup, a hoop or a cuff at the end of the measuring head, said cup, hoop or cuff being tightened around the finger depending on the size of the latter.

In yet another embodiment the measurement will be performed in a multi-point manner. For example, several light-guides can be carried to the band mounted around a forearm or a finger.

## Example 1. Determination $FM_{index}$ as a parameter of oscillatory function of microcirculation

NADH fluorescence from skin cells of the hand of a human subject and changes of intensity of this fluorescence (in arbitrary units) in time were measured.

The measurement was performed using prototype device equipped with a measuring head comprising of a light emitting diode LED, photodiode detector, interference filters in one arrangement attached to the armrest with the optical window suitable for measuring the emission from the surface of the skin.

The wavelength of the excitation light was 340 nm and the wavelength of monitored emission signal was 460 nm.

Intensity of a fluorescence signal from the hand of the subject freely placed on an armrest without any stimulation or blocking of circulation was collected and recorded for 5 minutes.

The measurements were performed in apparently healthy volunteers as a control as well as in the group of oncological patients. The course of florescence signal in time was recorded and plotted against time.

All results were normalized to the mean signal value in the between 2 and 3 minutes of measurement (central measurement period). The mean value of the results from this interval was computed and then fluorescence signals in the whole measurement range is divided by this mean value. In this manner normalized presentation of results is obtained (about the value 1), independently of an individual level of the fluorescence from the skin of a given subject.

For further steps results during first minute of the measurement were rejected. Fitting a baseline was performed for normalized results and mean squared errors (also called here Flow motion index $FM_{index}$) were determined on the basis of normalized results using computer program as a parameter of oscillatory function of skin microcirculation in a subject.

To obtain values in the range ones - hundreds calculated values od $FM_{index}$ were multiplied by $10^6$; obtained values were rounded to decimals.

Oscillatory function parameters $FM_{index}$ were calculated according to the following equation:
where:

SSE - residual sum of squares;

MSE - mean squared error = the residual sum of squares divided by the number of degrees of freedom;

n - the number of measurement points dependent on sampling frequency: here every 40 ms;

m - the number of regression parameters (here second order polynomial, i.e. m = 3)

The results are presented in Table 1 (control group of volunteers) and Table 2 (oncological patients). For some subjects several repeated measurements were performed and mean values were determined.

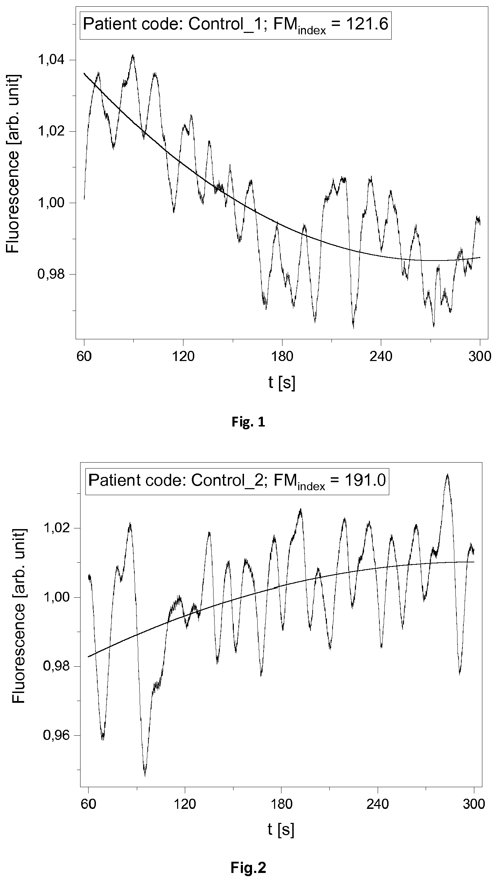

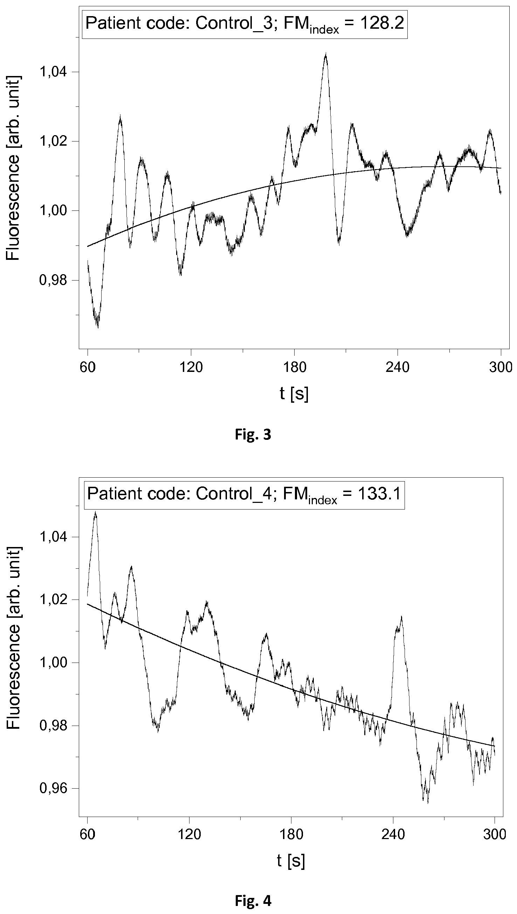

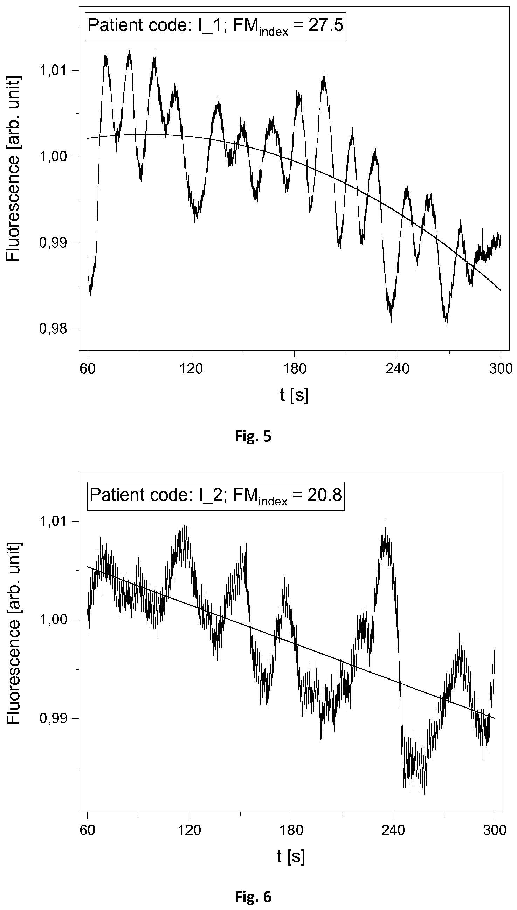

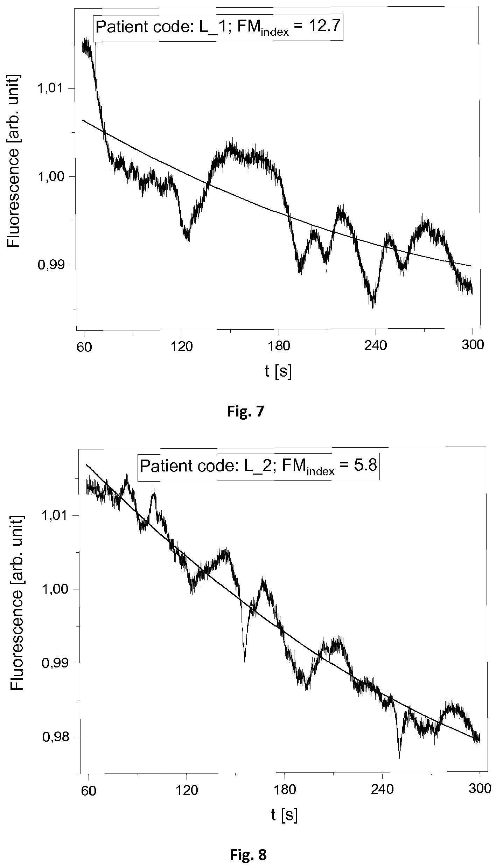

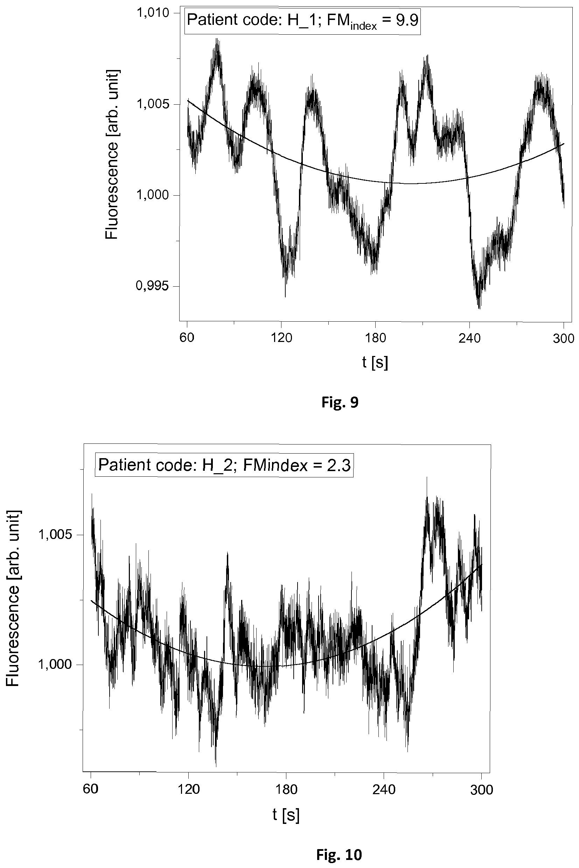

Additionally, time courses of the fluorescence signal with fitted baselines about which signals oscillated were plotted and presented on Figures 1 to 4 for control subjects 1, 2, 3 and 4, and in Figures 5 to 10 for patients I_1, I_2, L_1, L_2, H_1, and H_2, respectively.

Table 1. Healthy volunteers - control group

| Subject | $FM_{index}$ | | | | | | | mean |
|---|---|---|---|---|---|---|---|---|
| Control_1 | 121.6 | | | | | | | 121.6 |
| Control_2 | 204.8 | 194.6 | 211.0 | 191.0 | 117.9 | 257.6 | 285.7 | 208.9 |
| Control_3 | 128.2 | 138.6 | 63.1 | | | | | 110.0 |
| Control_4 | 221.7 | 133.1 | 60.1 | 26.1 | 155.4 | | | 119.3 |
| Control_5 | 96.1 | 134.7 | 286.1 | | | | | 172.3 |
| Control_6 | 39.3 | 39.4 | 42.9 | | | | | 40.5 |
| Control_7 | 53.7 | 82.2 | 52.2 | | | | | 62.7 |
| Control_8 | 104.0 | | | | | | | 104.0 |

The mean for the whole group was 117.4.

Table 2. Oncological patients

| Patient | $FM_{index}$ | Mean |
|---|---|---|
| Patients with intestine cancer | | |
| I_1 | 27.5 | |
| I_2 | 20.8 | |
| I_3 | 122.0 | |
| I_4 | 68.9 | |
| I_5 | 10.7 | |
| I_6 | 51.7 | |
| I_7 | 110.1 | 58.8 |
| Patients with lung cancer | | |
| L_1 | 12.7 | |
| L_2 | 5.8 | |
| L_3 | 46.0 | 21.5 |

(continued)

| Patients with head cancer | | |
|---|---|---|
| H_1 | 9.9 | |
| H_2 | 2.3 | |
| H_3 | 3.5 | |
| H_4 | 19.0 | 8.7 |

Statistical analysis: Student's t-distribution with two samples assuming unequal variances.

Variable 1 is the mean of the $FM_{index}$ for control group, Variable 2 is the $FM_{index}$ for the groups of patients.

1. Control versus patients with intestine cancer

| | Variable 1 | Variable 2 |
|---|---|---|
| Mean | 117.4125 | 58.814286 |
| Variance | 2935.36125 | 1919.2748 |
| Observations | 8 | 7 |
| Hypothesized Mean Difference | 0 | |
| df | 13 | |
| t Stat | 2.314305705 | |
| P(T<=t) one-tail | 0.018822754 | |
| T Critical one-tail | 1.770933396 | |
| P(T<=t) two-tail | 0.037645507 | |
| T Critical two-tail | 2.160368656 | |

2. Control versus patients with lung cancer

| | Variable 1 | Variable 2 |
|---|---|---|
| Mean | 117.4125 | 21.5 |
| Variance | 2935.36125 | 462.09 |
| Observations | 8 | 3 |
| Hypothesized Mean Difference | 0 | |
| df | 9 | |
| t Stat | 4.202203933 | |
| P(T<=t) one-tail | 0.001149589 | |
| T Critical one-tail | 1.833112933 | |
| P(T<=t) two-tail | 0.002299178 | |
| T Critical two-tail | 2.262157163 | |

3. Control versus patients with head cancer

| | Variable 1 | Variable 2 |
|---|---|---|
| Mean | 117.4125 | 8.675 |
| Variance | 2935.36125 | 58.509167 |

(continued)

|  | Variable 1 | Variable 2 |
|---|---|---|
| Observations | 8 | 4 |
| Hypothesized Mean Difference | 0 | |
| df | 8 | |
| t Stat | 5.566792934 | |
| P(T<=t) one-tail | 0.000265216 | |
| T Critical two-tail | 1.859548038 | |
| P(T<=t) one-tail | 0.000530433 | |
| T Critical two-tail | 2.306004135 | |

Despite of small number of tested subjects, virtually all analyses show statistically significant difference (decline of oscillations) with respect to control group. The method possesses significant diagnostic potential.

Example 2. Determination of skin flowmotion index ($FM_{index}$) in diabetic patients

Skin flowmotion index ($FM_{index}$) was determined in groups of diabetes mellitus 1 (DM1) and diabetes mellitus 2 (DM2) patients as an example of disease that impairs blood microcirculation and in control healthy groups of males and females of different age.

Clinical characteristic of the study population was as shown in Table 3 below.

Table 3.

| Characteristics | Control (DM1) | DM1 | Control (DM2) | DM2 |
|---|---|---|---|---|
| | n = 47 | n = 37 | n = 22 | n = 70 |
| Age [years] | 39.1 ± 5.4 | 39.7 ± 4.4 | 62.3 ± 7.6 | 63.0 ± 8.1 |
| Female/Male | 22/ 25 | 21/ 16 | 12/ 10 | 32/38 |
| BMI [kg/m2] | 24.7 ± 4.2 | 26.8 ± 4.2 | 26.2 ± 3.6 | 31.4 ± 5.5 |
| Disease duration [years] | - | 19.5 ± 7.9 | - | 14.6 ± 7.4 |
| Smoking | 2 (4.3) | 4 (10.8) | 1 (4.5) | 14 (20.0) |
| Characteristics | Control (DM1) | DM1 | Control (DM2) | DM2 |
| Hypertension | - | 12 (32.4) | - | 65 (92.9) |
| Prevalent CVD | - | 1 (2.7) | - | 31 (44.3) |
| Retinopathy | - | 14 (37.8) | - | 27 (38.6) |
| Neuropathy | - | 7 (18.9) | - | 15 (21.4) |
| Nephropathy | - | 4 (10.8) | - | 6 (8.6) |
| Diabetic foot | - | 4 (10.8) | - | 24 (34.3) |
| HbA1c [%] | - | 8.3 ± 1.5 | - | 8.2 ± 1.4 |

Continuous variables, mean ± SD; dichotomous variables, no. (%);
CVD - cardiovascular disease (ischemic heart disease, heart failure, myocardial infraction, stroke)

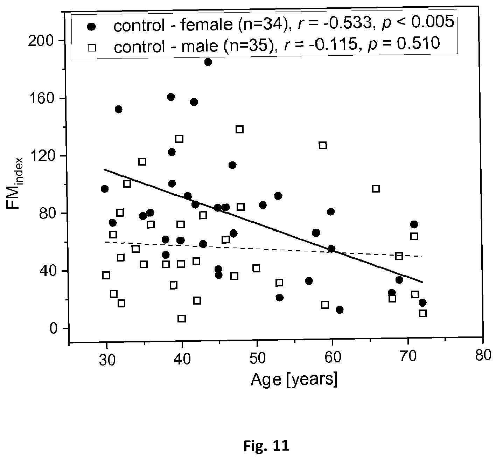

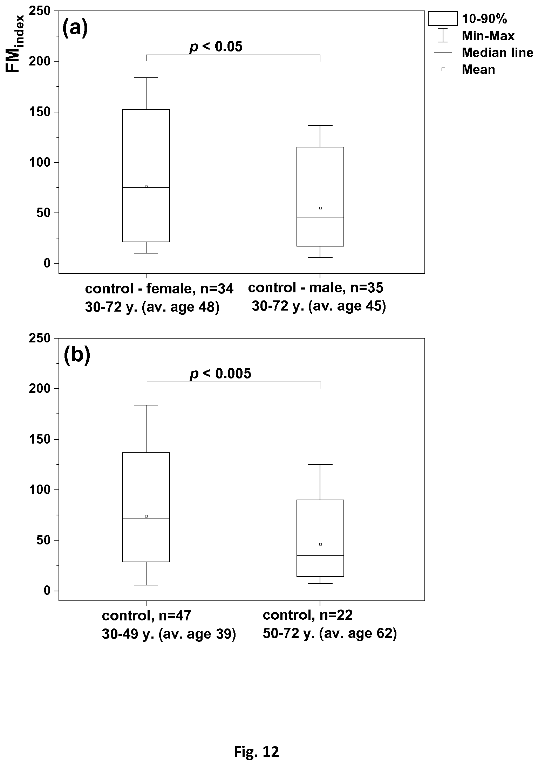

Correlation of determined $FM_{index}$ with age for control groups is shown in Fig. 11, wherein solid line corresponds to linear fit to black circles (females) and dotted line corresponds to linear fit to open squares (males). Comparison of $FM_{index}$ for control groups with respect to sex and age is shown in Fig. 12a, and in Fig. 12b for the whole control group of males and females together divided into subgroups of various age. It can be clearly seen that in the whole age range women have significantly better $FM_{index}$ than males (Fig. 12a). It can be also seen (Fig. 12b) that younger population (30-49 years, males and females together) had significantly higher $FM_{index}$ values that older population (50-72 years).

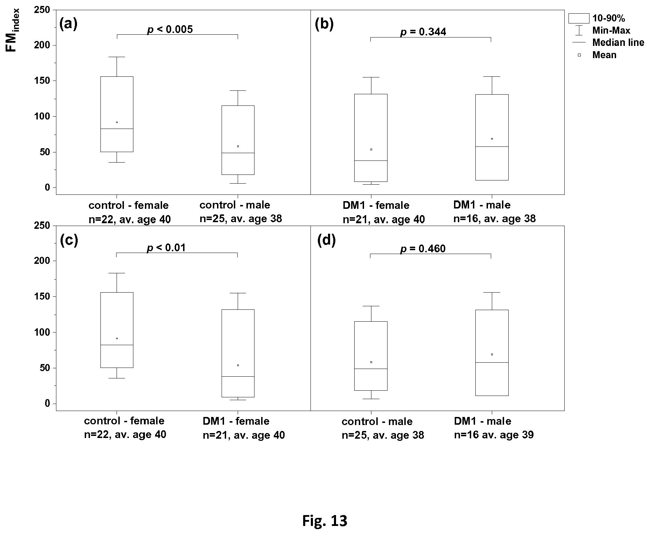

Comparison of $FM_{index}$ for group of diabetic patients with diabetes mellitus 1 (DM1) with respect to sex and age is shown in Fig. 13. It has been shown that the presence of a pathology completely blurs sex differences (Fig. 13a and 13b). On the other hand $FM_{index}$ excellently differentiates between young women with DM1 and young control groups (Fig. 13c). There is no such difference for male subgroup (Fig. 13d).

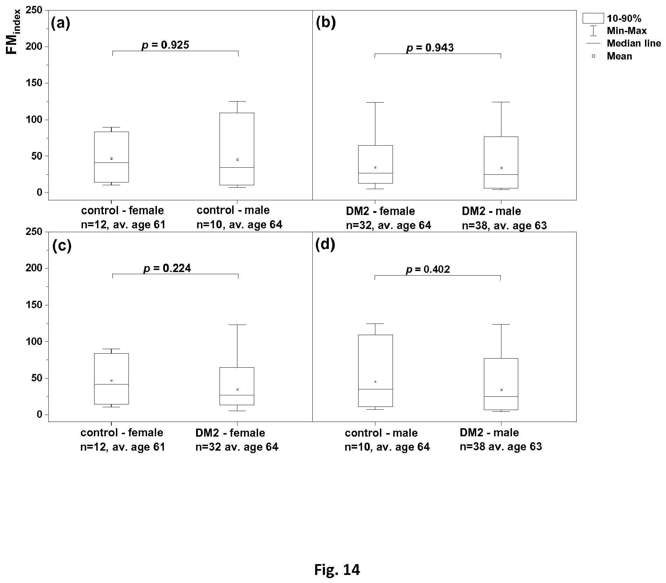

Comparison of $FM_{index}$ for group of diabetic patients with diabetes mellitus 2 (DM2) with respect to sex and age is shown in Fig. 14. No significant differences can be seen, since $FM_{index}$ parameter declines with age and this parameter loses its sensitivity.

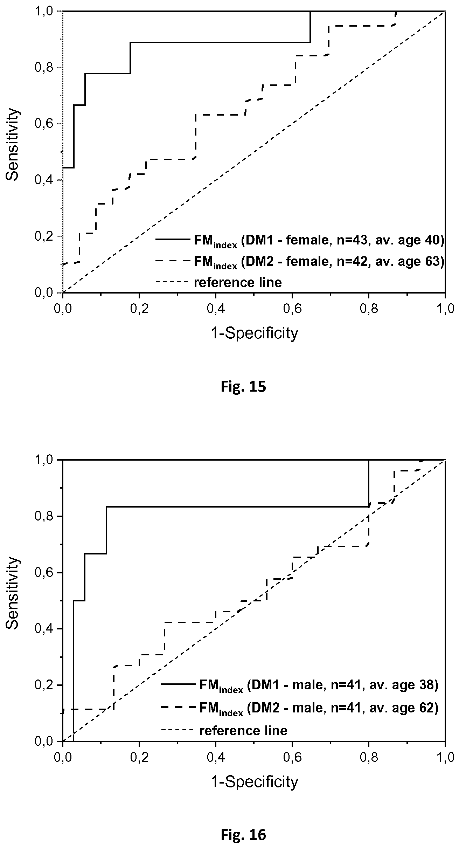

ROC (Receiver Operating Characteristics) AUC (Area Under Curve) curves for female diabetic patients in groups DM1 and DM2 of the age below and above 60 years as presented in Fig. 15 show high diagnostic power and value of $FM_{index}$ parameter for assessment of microvascular complications in diabetes (retinopathy, neuropathy, nephropathy and diabetic foot). High diagnostic value can be seen in the group of females with DM1 (age below 60 years), however there is no significant diagnostic potential for DM2 group, mainly because $FM_{index}$ parameter is low in the age above 60 years.

Statistical data for curves presented in Fig. 15 are presented in Table 4 below.

Table 4

| Parameter | ROC curve (AUC) | Standard error | 95 % Confidence interval | Significance level *p* |
|---|---|---|---|---|
| $FM_{index}$ (DM1 - female, P/N = 9/34) | 0.895 | 0.052 | 0.793 - 0.998 | <0.0005 |
| $FM_{index}$ (DM2 -female, P/N = 19/23) | 0.668 | 0.084 | 0.504 - 0.832 | 0.063 |

* P - positive (at least one vascular complication out ot tour (retinopathy, nephropathy, neuropathy and diabetic foot));
N - negative (without vascular complications)

Fig. 16 shows ROC (AUC) curves for male diabetic patients in groups DM1 and DM2.

Statistical data for curves presented in Fig. 16 are presented in Table 5 below.

Table 5.

| Parameter | ROC curve (AUC) | Standard error | 95% Confidence interval | Significance level *p* |
|---|---|---|---|---|
| $FM_{index}$ (DM1 - male, P/N = 6/35) | 0.824 | 0.086 | 0.655 - 0.993 | <0.05 |
| $FM_{index}$ (DM2 - male, P/N = 26/15) | 0.536 | 0.097 | 0.346 - 0.726 | 0.705 |

* P - positive (at least one vascular complication out of four (retinopathy, nephropathy, neuropathy and diabetic foot));
N - negative (without vascular complications)

Although research on a bigger population would be required to determine limiting values of $FM_{index}$, it has been calculated by the present inventors on the basis of preliminary studies that respective values for estimation of the risk of microvascular complications in DM1 patients as an example of a pathology are as presented in Table 6 below.

Table 6

| Sex | $FM_{index}$ | | |
|---|---|---|---|
| | Low | Risk Moderate | High |
| Female | > 70 | 30 - 70 | < 30 |
| Male | > 60 | 20 - 60 | < 20 |

**Claims**

1. A method of detection of the presence of a pathology that impairs blood microcirculation or a risk of said pathology, in a female human subject of the age below 60 years, by determination of a parameter of oscillatory function of the skin microcirculation in said female subject, said method comprising the following steps:

   - illumination of a selected location on non-hairy skin of said female human subject in a resting steady condition without any blockage or stimulation of the blood flow with exciting light capable of inducing skin NADH fluorescence signal for a period of time;
- simultaneously with said illumination detecting, measuring and recording as a function of time an induced NADH fluorescence signal emitted from the said selected location to obtain the time course of the NADH fluorescence signal intensity in the said period of time; and
- computer implemented steps of:

- fitting of a baseline about which the NADH fluorescence signal oscillates to the said time course of the NADH fluorescence signal intensity; and
- determination of a mean squared error of the deviation of the NADH fluorescence signal from the fitted baseline as the parameter of oscillatory function of skin microcirculation.

2. A method of determination of a parameter for detection of the presence of a pathology that impairs blood microcirculation or a risk of said pathology, in a female human subject of the age below 60 years, wherein said parameter is determined as a parameter of oscillatory function of skin microcirculation in the following steps:

- illumination of a selected location on non-hairy skin of said female human subject in a resting steady condition without any blockage or stimulation of the blood flow with exciting light capable of inducing skin NADH fluorescence signal for a period of time;
- simultaneously with said illumination detecting, measuring and recording as a function of time the induced NADH fluorescence signal emitted from the said selected location to obtain the time course of the NADH fluorescence signal intensity in the said period of time; and
- computer implemented steps of:

- fitting of a baseline about which the NADH fluorescence signal oscillates to the said time course of the NADH fluorescence signal; and
- determination of a mean squared error of the deviation of the NADH fluorescence signal from the fitted baseline as the parameter of oscillatory function of skin microcirculation.

3. The method of claim 1 or 2, wherein said detection is the early detection.

4. The method of any one of claims 1 to 3, wherein said pathology is selected from the group consisting of diabetes and its complications, including diabetes mellitus type 1 and diabetes mellitus type 2, metabolic syndrome, cardiovascular disease, including coronary arterial disease, idiopathic hypertension and stroke, cancer, neurodegenerative diseases, autoimmunological diseases, depression, chronic renal failure and bad abidance of menopause.

5. The method of any one of claims 1 to 4, said fitting of the baseline is performed using polynomial regression method.

6. The method of claim 5, wherein second order polynomial regression method is used.

7. The method of claim 5 or 6, wherein said fitting is performed using least squares method.

8. The method of any one of claims 1 to 7, wherein said period of time is at least 1 minute.

9. The method of any one of claims 1 to 8, wherein for said fitting of the baseline an initial period of measurement is rejected.

10. A device or system for determination of a parameter for detection of the presence of a pathology that impairs blood microcirculation or risk of said pathology, in a female human subject of the age below 60 years, said parameter being determined as a parameter of oscillatory function of skin microcirculation, the device or system consisting of:

- a means for illumination of a skin of said female human subject with exciting light capable to induce NADH fluorescence;
- a means for detecting and measuring NADH fluorescence signal emitted from the skin;
- a processing unit which is configured to perform the following:

- receiving and recording time course of said intensity of the NADH fluorescence signal,
- fitting of a baseline about which the NADH fluorescence signal oscillates to the said time course of the fluorescence signal, and

- determination a mean squared error of the deviation of the NADH fluorescence signal from the fitted baseline as said parameter of oscillatory function of skin microcirculation,

and
- a means for displaying a result of determination of the said parameter of oscillatory function of skin microcirculation.

11. The device or system according claim 10 further comprising a means for displaying the fluorescence signal oscillations against time and its pattern analysis or for retrieving and saving the said course of the fluorescence signal against time for it to be printed and its pattern analyzed later.

12. The device according to claim 10 or 11, wherein said fitting of the baseline is performed using polynomial regression method.

13. The device according to claim 12, wherein second order polynomial regression method is used.

14. The device according to claim 12 or 13, wherein least squares method is used.

**Fig. 1**

**Fig.2**

Fig. 3

Fig. 4

**Fig. 5**

**Fig. 6**

**Fig. 7**

**Fig. 8**

Patient code: H_1; $FM_{index}$ = 9.9

**Fig. 9**

Patient code: H_2; FMindex = 2.3

**Fig. 10**

Fig. 11

Fig. 12

**Fig. 13**

**Fig. 14**

Fig. 15

Fig. 16

## EUROPEAN SEARCH REPORT

Europäisches Patentamt
European Patent Office
Office européen des brevets

Application Number

EP 19 46 1568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARCIN HELLMANN ET AL: "Reproducibility of flow mediated skin fluorescence to assess microvascular function", MICROVASCULAR RESEARCH., vol. 113, 18 May 2017 (2017-05-18), pages 60-64, XP55665164, US ISSN: 0026-2862, DOI: 10.1016/j.mvr.2017.05.004 * sections 2.2., 2.3., 2.4., 2.8.; figures 1, 2 * | 1-14 | INV. A61B5/026 |
| X | KATARZYNSKA JOANNA ET AL: "Flow Mediated Skin Fluorescence technique reveals remarkable effect of age on microcirculation and metabolic regulation in type 1 diabetes", MICROVASCULAR RESEARCH, vol. 124, 23 February 2019 (2019-02-23), pages 19-24, XP085661031, ISSN: 0026-2862, DOI: 10.1016/J.MVR.2019.02.005 * sections 2.2, 2.3; figures 1, 2 * | 1-14 | |
| X | US 2012/310057 A1 (GEBICKI JERZY [PL] ET AL) 6 December 2012 (2012-12-06) * paragraphs [0023] - [0076]; figure 1 * | 1-14 | TECHNICAL FIELDS SEARCHED (IPC) A61B |

-/--

The present search report has been drawn up for all claims

| Place of search | Date of completion of the search | Examiner |
|---|---|---|
| The Hague | 7 February 2020 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 19 46 1568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MARIA TARNAWSKA ET AL: "A pilot study with flow mediated skin fluorescence: A novel device to assess microvascular endothelial function in coronary artery disease", CARDIOLOGY JOURNAL, vol. 25, no. 1, 27 February 2018 (2018-02-27), pages 120-127, XP055510420, PL ISSN: 1897-5593, DOI: 10.5603/CJ.a2017.0096 * sections "Study design", "Technical description of FMSF device", "Data analysis"; figures 1-3 * | 1-14 | |
| X,D | PIOTROWSKI L ET AL: "Note: Flow mediated skin fluorescence-A novel technique for evaluation of cutaneous microcirculation", REVIEW OF SCIENTIFIC INSTRUMENTS, AIP, MELVILLE, NY, US, vol. 87, no. 3, 30 March 2016 (2016-03-30), XP012211286, ISSN: 0034-6748, DOI: 10.1063/1.4945044 [retrieved on 1901-01-01] * the whole document * | 1-14 | TECHNICAL FIELDS SEARCHED (IPC) |
| E | WO 2019/206869 A1 (ANGIONICA SP Z O O [PL]) 31 October 2019 (2019-10-31) * the whole document * | 10-14 | |

The present search report has been drawn up for all claims

| Place of search | Date of completion of the search | Examiner |
|---|---|---|
| The Hague | 7 February 2020 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 46 1568

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2020

| Patent document cited in search report | Publication date | Patent family member(s) | Publication date |
|---|---|---|---|
| US 2012310057 A1 | 06-12-2012 | NONE | |
| WO 2019206869 A1 | 31-10-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012164494 A

### Non-patent literature cited in the description

- **H. NILLSON ; C. AALKJÆR.** Vasomotion: Mechanisms and Physiological Importance. *Molecular Interventions,* 2003, vol. 3 (2), 79-89
- **V. L. CLIFTON et al.** Microvascular effects of corticotropin-releasing hormone in human skin vary in relation to estrogen concentration during the menstrual cycle. *Journal of Endocrinology,* 2005, vol. 186, 69-76
- **A. VINET et al.** Impact of a Lifestyle Program on Vascular Insulin Resistance in Metabolic Syndrome Subjects: The RESOLVE Study. *The Journal of Clinical Endocrinology and Metabolism,* 2015, vol. 100 (2), 442-450
- **STEWART, J. et al.** Noninvasive interrogation of microvasculature for signs of endothelial dysfunction in patients with chronic renal failure. *American Journal of Physiology - Heart and Circulatory Physiology,* 2004, vol. 287, H2687-H2696
- **M. ROSSI.** Diagnostic values of skin vasomotion investigation in vascular diseases. *Advances in Biomedical Research,* 2010, 374-380
- **M. HELLMAN et al.** *Microvascular Research, Reproducibility of flow mediated skin fluorescence to assess microvascular function,* 2017, vol. 113, 60-84
- **L. PIOTROWSKI et al.** Flow mediated skin fluorescence - A novel technique for evaluation of cutaneous microcirculation. *Review of Scientific Instruments,* 2016, vol. 87 (3), 036111
- **M. TARNAWSKA.** A pilot study with flow mediated skin fluorescence: A novel device to assess microvascular endothelial function in coronary artery disease. *Cardiology Journal,* 2018, vol. 25 (2), 120-127